# EUROPEAN PATENT SPECIFICATION

(11) **EP 2 613 795 B1**
(45) Date of publication and mention of the grant of the patent: **26.10.2016**
(21) Application number: 11824038.1
(22) Date of filing: 07.09.2011
(51) Int. Cl.: A61K 31/496, A61P 43/00, A61P 27/02

(54) **DIKETOPIPERAZINES FOR TREATING VASCULAR HYPERPERMEABILITY**
DIKETOPIPERAZINE ZUR BEHANDLUNG DER VASKULÄREN HYPERPERMEABILITÄT
DIKETOPIPERAZINES POUR TRAITER L'HYPERPERMÉABILITÉ VASCULAIRE

(30) Priority: 07.09.2010 US 380404 P
(43) Date of publication of application: 17.07.2013
(73) Proprietor: Ampio Pharmaceuticals, Inc., Englewood, CO 80112 (US)
(72) Inventor: BAR-OR, David, Englewood CO 80110 (US)
(74) Representative: Green, Mark Charles
(86) International application number: PCT/US2011/050612
(87) International publication number: WO 2012/033789

(56) References cited:
- WO-A1-2009/042193
- WO-A1-2009/146320
- WO-A2-2004/103304
- WO-A2-2012/033789
- US-A1- 2002 052 381
- SCHLINGEMANN ET AL.: 'Role of vascular permeability factor/vascular endothelial growth factor in eye disease' BR J OPHTHALMOL vol. 81, 1997, pages 501 - 512, XP008162953
- SAMANTA ET AL.: 'Crystal Structure of Human Plasma Platelet-activating Factor Acetylhydrolase' JOURNAL OF BIOLOGICAL CHEMISTRY vol. 283, no. 46, 14 November 2008, pages 31617 - 31624, XP008149417
- RAMÍREZ M M ET AL: "Platelet activating factor modulates microvascular permeability through nitric oxide synthesis.", MICROVASCULAR RESEARCH SEP 1995, vol. 50, no. 2, September 1995 (1995-09), pages 223-234, ISSN: 0026-2862
- NORIHIKO SHIMAZAKI ET AL: "PAF inhibitory activity of diketopiperazines: Structure-activity relationships", LIPIDS, SPRINGER-VERLAG, BERLIN/HEIDELBERG, vol. 26, no. 12, 1 December 1991 (1991-12-01), pages 1175-1178, XP035173964, ISSN: 1558-9307, DOI: 10.1007/BF02536526

## Description

### FIELD OF THE INVENTION

The invention relates to a method and kit for inhibiting vascular hyperpermeability and the edema and other adverse effects that result from it. The invention also relates to a method and kit for modulating the cytoskeleton of endothelial cells. Both methods comprise administering to an animal a diketopiperazine (DKP) according to the appended claims or a pharmaceutically-acceptable salt of either one of them.

### BACKGROUND

The vascular endothelium lines the inside of all blood vessels. It acts as the interface between the blood and the tissues and organs. The endothelium forms a semipermeable barrier that maintains the integrity of the blood fluid compartment, but permits passage of water, ions, small molecules, macromolecules and cells in a regulated manner. Dysregulation of this process produces vascular leakage into underlying tissues. Leakage of fluid into tissues causing edema can have serious and life threatening consequences in a variety of diseases. Accordingly, it would be highly desirable to have a method for reducing edema, preferably at its earliest stage, and restoring the endothelial barrier to physiological.

### SUMMARY OF THE INVENTION

The invention relates to such a method. In particular, the invention relates to a method of inhibiting vascular hyperpermeability and the edema and other adverse effects that result from it. The method comprises administering to an animal in need thereof an effective amount of an active ingredient, wherein the active ingredient comprises a diketopiperazine, selected from DA-DKP, MR-DKP and YE-DKP, or a pharmaceutically-acceptable salt of either one of them.

Inhibition of vascular hyperpermeability according to the invention includes inhibition of paracellular-caused hyperpermeability and transcytosis-caused hyperpermeability. Recent evidence indicates that transcytosis-caused hyperpermeability is the first step of a process that ultimately leads to tissue and organ damage in many diseases and conditions. Accordingly, the present invention provides a means of early intervention in these diseases and conditions which can reduce, delay or even potentially prevent the tissue and organ damage seen in them.

The present disclosure also relates to a method of modulating the cytoskeleton of endothelial cells in an animal. The method comprises administering an effective amount of an active ingredient, wherein the active ingredient comprises a diketopiperazine selected from DA-DKP. MR-DKP and YE-DKP, or a pharmaceutically-acceptable salt of either one of them, to the animal.

The invention further relates to a kit. The kit comprises the diketopiperazines according to appended claim 1 or a pharmaceutically-acceptable salt of either of them to the animal.

"Vascular hyperpermeability" is used herein to mean permeability of a vascular endothelium that is increased as compared to basal levels. "Vascular hyperpermeability," as used herein, includes paracellular-caused hyperpermeability and transcytosis-caused hyperpermeability.

"Paracellular-caused hyperpermeability" is used herein to mean vascular hyperpermeability caused by paracellular transport that is increased as compared to basal levels. Other features of "paracellular-caused hyperpermeability" are described below.

"Paracellular transport" is used herein to mean the movement of ions, molecules and fluids through the interendothelial junctions (IEJs) between the endothelial cells of an endothelium.

"Transcytosis-caused hyperpermeability" is used herein to mean vascular hyperpermeability caused by transcytosis that is increased as compared to basal levels.

"Transcytosis" is used herein to mean the active transport of macromolecules and accompanying fluid-phase plasma constituents across the endothelial cells of the endothelium. Other features of "transcytosis" are described below.

"Basal level" is used herein to refer to the level found in a normal tissue or organ.

"Inhibiting, "inhibit" and similar terms are used herein to mean to reduce, delay or prevent.

An animal is "in need of" treatment according to the invention if the animal presently has a disease or condition mediated by vascular hyperpermeability, exhibits early signs of such a disease or condition, or has a predisposition to develop such a disease or condition.

"Mediated" and similar terms are used here to mean caused by, causing, involving or exacerbated by, vascular hyperpermeability.

### DETAILED DESCRIPTION OF THE PRESENTLY-

### PREFERRED EMBODIMENTS OF THE INVENTION

The endothelium is a key gatekeeper controlling the exchange of molecules from the blood to the tissue parenchyma. It largely controls the permeability of a particular vascular bed to blood-borne molecules. The permeability and selectivity of the endothelial cell barrier is strongly dependent on the structure and type of endothelium lining the microvasculature in different vascular beds. Endothelial cells lining the microvascular beds of different organs exhibit structural differentiation that can be grouped into three primary morphologic categories: sinusoidal, fenestrated and continuous.

Sinusoidal endothelium (also referred to as "discontinuous endothelium") has large intercellular and intracellular gaps and no basement membrane, allowing for minimally restricted transport of molecules from the capillary lumen into the tissue and vice versa. Sinusoidal endothelium is found in liver, spleen and bone marrow.

Fenestrated endothelia are characterized by the presence of a large number of circular transcellular openings called fenestrae with a diameter of 60 to 80 nm. Fenestrated endothelia are found in tissues and organs that require rapid exchange of small molecules, including kidney (glomeruli, peritubular capillaries and ascending vasa recta), pancreas, adrenal glands, endocrine glands and intestine. The fenestrae are covered by thin diaphragms, except for those in mature, healthy glomeruli. See Ichimura et al., J. Am. Soc. Nephrol., 19:1463-1471 (2008).

Continuous endothelia do not contain fenestrae or large gaps. Instead, continuous endothelia are characterized by an uninterrupted endothelial cell monolayer. Most endothelia in the body are continuous endothelia, and continuous endothelium is found in, or around, the brain (blood brain barrier), diaphragm, duodenal musculature, fat, heart, some areas of the kidneys (papillary microvasculature, descending vasa recta), large blood vessels, lungs, mesentery, nerves, retina (blood retinal barrier), skeletal muscle, testis and other tissues and organs of the body.

Endothelial transport in continuous endothelium can be thought of in a general sense as occurring by paracellular and transcellular pathways. The paracellular pathway is the pathway between endothelial cells, through the interendothelial junctions (IEJs). In unperturbed continuous endothelium, water, ions and small molecules are transported paracellularly by diffusion and convection. A significant amount of water (up to 40%) also crosses the endothelial cell barrier transcellularly through water-transporting membrane channels called aquaporins. A variety of stimuli can disrupt the organization of the IEJs, thereby opening gaps in the endothelial barrier. The formation of these intercellular gaps allows passage of fluid, ions, macromolecules (e.g., proteins) and other plasma constituents between the endothelial cells in an unrestricted manner. This paracellular-caused hyperpermeability produces edema and other adverse effects that can eventually result in damage to tissues and organs.

The transcellular pathway is responsible for the active transport of macromolecules, such as albumin and other plasma proteins, across the endothelial cells, a process referred to as "transcytosis." The transport of macromolecules occurs in vesicles called caveolae. Almost all continuous endothelia have abundant caveolae, except for continuous endothelia located in brain and testes which have few caveolae. Transcytosis is a multi-step process that involves successive caveolae budding and fission from the plasmalemma and translocation across the cell, followed by docking and fusion with the opposite plasmalemma, where the caveolae release their contents by exocytosis into the interstitium. Transcytosis is selective and tightly regulated under normal physiological conditions.

There is a growing realization of the fundamental importance of the transcellular pathway. Transcytosis of plasma proteins, especially albumin which represents 65% of plasma protein, is of particular interest because of its ability to regulate the transvascular oncotic pressure gradient. As can be appreciated, then, increased transcytosis of albumin and other plasma proteins above basal levels will increase the tissue protein concentration of them which, in turn, will cause water to move across the endothelial barrier, thereby producing edema.

Low density lipoproteins (LDL) are also transported across endothelial cells by transcytosis. In hyperlipidemia, a significant increase in transcytosis of LDL has been detected as the initial event in atherogenesis. The LDL accumulates in the subendothelial space, trapped within the expanded basal lamina and extracellular matrix. The subendothelial lipoprotein accumulation in hyperlipidema is followed by a cascade of events resulting in atheromatous plaque formation. Advanced atherosclerotic lesions are reported to be occasionally accompanied by the opening of IEJs and massive uncontrolled passage of LDL and albumin.

Vascular complications are a hallmark of diabetes. At the level of large vessels, the disease appears to be expressed as an acceleration of an atherosclerotic process. With respect to microangiopathy, alterations in the microvasculature of the retina, renal glomerulus and nerves cause the greatest number of clinical complications, but a continuously increasing number of investigations show that diabetes also affects the microvasculature of other organs, such as the mesentery, skin, skeletal muscle, heart, brain and lung, causing additional clinical complications. In all of these vascular beds, changes in vascular permeability appear to represent a hallmark of the diabetic endothelial dysfunction.

In continuous endothelium, capillary hyperpermeability to plasma macromolecules in the early phase of diabetes is explained by an intensification of transendothelial vesicular transport (*i.e*., by increased transcytosis) and not by the destabilization of the IEJs. In addition, the endothelial cells of diabetics, including those of the brain, have been reported to contain an increased number of caveolae as compared to normals, and glycated proteins, particularly glycated albumin, are taken up by endothelial cells and transcytosed at substantially greater rates than their native forms. Further, increased transcytosis of macromolecules is a process that continues beyond the early phase of diabetes and appears to be a cause of edema in diabetic tissues and organs throughout the disease if left untreated. This edema, in turn, leads to tissue and organ damage. Similar increases in transcellular transport of macromolecules have been reported in hypertension.

Paracellular-caused hyperpermeability is also a factor in diabetes and the vascular complications of diabetes. The IEJs of the paracellular pathway include the adherens junctions (AJs) and tight junctions (TJs). Diabetes alters the content, phosphorylation and localization of certain proteins in both the AJs and TJs, thereby contributing to increased endothelial barrier permeability.

In support of the foregoing discussion and for further information, see Frank et al., Cell Tissue Res., 335:41-47 (2009), Simionescu et al., Cell Tissue Res., 335:27-40 (2009); van den Berg et al., J. Cost. Fibros., 7(6): 515-519 (2008); Viazzi et al., Hypertens. Res., 31:873-879 (2008); Antonetti et al., Chapter 14, pages 340-342, in Diabetic Retinopathy (edited by Elia J. Duh, Humana Press, 2008), Felinski et al., Current Eye Research, 30:949-957 (2005), Pascariu et al., Journal of Histochemistry & Cytochemistry, 52(1):65-76 (2004); Bouchard et al., Diabetologia, 45:1017-1025 (2002); Arshi et al., Laboratory Investigation, 80(8):1171-1184 (2000); Vinores et al., Document Ophthalmologica, 97:217-228 (1999); Oomen et al., European Journal of Clinical Investigation, 29:1035-1040 (1999); Vinores et al., Pathol. Res. Pract., 194:497-505 (1998); Antonetti et al., Diabetes, 47:1953-1959 (1998), Popov et al., Acta Diabetol., 34:285-293 (1997); Yamaji et al., Circulation Research, 72:947-957 (1993); Vinores et al., Histochemical Journal, 25:648-663 (1993); Beals et al., Microvascular Research, 45:11-19 (1993); Caldwell et al., Investigative Ophthalmol. Visual Sci., 33(5):16101619 (1992).

Endothelial transport in fenestrated endothelium also occurs by transcytosis and the paracellular pathway. In addition, endothelial transport occurs by means of the fenestrae. Fenestrated endothelia show a remarkably high permeability to water and small hydrophilic solutes due to the presence of the fenestrae.

The fenestrae may or may not be covered by a diaphragm. The locations of endothelium with diaphragmed fenestrae include endocrine tissue (e.g., pancreatic islets and adrenal cortex), gastrointestinal mucosa and renal peritubular capillaries. The permeability to plasma proteins of fenestrated endothelium with diaphragmed fenestrae does not exceed that of continuous endothelium.

The locations of endothelium with nondiaphragmed fenestrae include the glomeruli of the kidneys. The glomerular fenestrated endothelium is covered by a glycocalyx that extends into the fenestrae (forming so-called "seive plugs") and by a more loosely associated endothelial cell surface layer of glycoproteins. Mathematical analyses of functional permselectivity studies have concluded that the glomerular endothelial cell glycocalyx, including that present in the fenestrae, and its associated surface layer account for the retention of up to 95% of plasma proteins within the circulation.

Loss of fenestrae in the glomerular endothelium has been found to be associated with proteinuria in several diseases, including diabetic nephropathy, transplant glomerulopathy, pre-eclampsia, diabetes, renal failure, cyclosporine nephropathy, serum sickness nephritis and Thy-1 nephritis. Actin rearrangement and, in particular, depolymerization of stress fibers have been found to be important for the formation and maintenance of fenestrae.

In support of the foregoing discussion of fenestrated endothelia and for additional information, see Satchell et al., Am. J. Physiol. Renal Physiol., 296:F947-F956 (2009); Haraldsson et al., Curr. Opin. Nephrol. Hypertens., 18:331-335 (2009); Ichimura et al., J. Am. Soc. Nephrol., 19:1463-1471 (2008); Ballermann, Nephron Physiol., 106:19-25 (2007); Toyoda et al., Diabetes, 56:2155-2160 (2007); Stan, "Endothelial Structures Involved In Vascular Permeability," pages 679-688, Endothelial Biomedicine (ed. Aird, Cambridge University Press, Cambridge, 2007); Simionescu and Antohe, "Functional Ultrastructure of the Vascular Endothelium: Changes in Various Pathologies," pages 42-69, The Vascular Endothelium I (eds. Moncada and Higgs, Springer-Verlag, Berlin, 2006).

Endothelial transport in sinusoidal endothelium occurs by transcytosis and through the intercellular gaps (interendothelial slits) and intracellular gaps (fenestrae). Treatment of sinusoidal endothelium with actin filament-disrupting drugs can induce a substantial and rapid increase in the number of gaps, indicating regulation of the porosity of the endothelial lining by the actin cytoskeleton. Other cytoskeleton altering drugs have been reported to change the diameters of fenestrae. Therefore, the fenestrae-associated cytoskeleton probably controls the important function of endothelial filtration in sinusodial endotheluium. In liver, defenestration (loss of fenestrae), which causes a reduction in permeability of the endothelium, has been associated with the pathogenesis of several diseases and conditions, including aging, atherogenesis, atherosclerosis, cirrhosis, fibrosis, liver failure and primary and metastatic liver cancers. In support of the foregoing and for additional information, see Yokomori, Med. Mol. Morphol., 41:1-4 (2008); Stan, "Endothelial Structures Involved In Vascular Permeability," pages 679-688, Endothelial Biomedicine (ed. Aird, Cambridge University Press, Cambridge, 2007); DeLeve, "The Hepatic Sinusoidal Endothelial Cell," pages 1226-1238, Endothelial Biomedicine (ed. Aird, Cambridge University Press, Cambridge, 2007); Pries and Kuebler, "Normal Endothelium," pages 1-40, The Vascular Endothelium I (eds. Moncada and Higgs, Springer-Verlag, Berlin, 2006); Simionescu and Antohe, "Functional Ultrastructure of the Vascular Endothelium: Changes in Various Pathologies," pages 42-69, The Vascular Endothelium I (eds. Moncada and Higgs, Springer-Verlag, Berlin, 2006); Braet and Wisse, Comparative Hepatology, 1:1-17 (2002); Kanai et al., Anat. Rec., 244:175-181 (1996); Kempka et al., Exp. Cell Res., 176:38-48 (1988); Kishimoto et al., Am. J. Anat., 178:241-249 (1987).

The present invention is defined by the appended claims. The invention relates to a method of inhibiting vascular hyperpermeability present in any tissue or organ containing or surrounded by continuous endothelium. As noted above, continuous endothelium is present in, or around, the brain (blood brain barrier), diaphragm, duodenal musculature, fat, heart, some areas of the kidneys (papillary microvasculature, descending vasa recta), large blood vessels, lungs, mesentery, nerves, retina (blood retinal barrier), skeletal muscle, skin, testis, umbilical vein and other tissues and organs of the body. Preferably, the continuous endothelium is that found in or around the brain, heart, lungs, nerves or retina.

The invention also relates to a method of inhibiting vascular hyperpermeability present in any tissue or organ containing or surrounded by fenestrated endothelium. As noted above, fenestrated endothelium is present in, or around, the kidney (glomeruli, peritubular capillaries and ascending vasa recta), pancreas, adrenal glands, endocrine glands and intestine. Preferably, the fenestrated endothelium is that found in the kidneys, especially that found in the glomeruli of the kidneys.

Further, any disease or condition mediated by vascular hyperpermeability can be treated by the method herein disclosed to inhibit the vascular hyperpermeability. Such diseases and conditions include diabetes, hypertension and atherosclerosis.

In particular, the vascular complications of diabetes, including those of the brain, heart, kidneys, lung, mesentery, nerves, retina, skeletal muscle, skin and other tissues and organs containing continuous or fenestrated endothelium, can be treated by the present invention. These vascular complications include edema, accumulation of LDL in the subendothelial space, accelerated atherosclerosis, and the following: brain (accelerated aging of vessel walls), heart (myocardial edema, myocardial fibrosis, diastolic dysfunction, diabetic cardiomyopathy), kidneys (diabetic nephropathy), lung (retardation of lung development in the fetuses of diabetic mothers, alterations of several pulmonary physiological parameters and increased susceptibility to infections), mesentery (vascular hyperplasy), nerves (diabetic neuropathy), retina (macular edema and diabetic retinopathy) and skin (redness, discoloration, dryness and ulcerations). Vascular hyperpermeability in both Type 1 (autoimmune) and Type 2 (non-insulin-dependent) diabetes can be inhibited by the method of the invention. Type 2 is the most common type of diabetes, affecting 90-95% of diabetics, and its treatment, especially the treatment of those with early signs of, or a predisposition to develop, Type 2 diabetes (see below), should be particularly beneficial.

Diabetic retinopathy is a leading cause of blindness that affects approximately 25% of the estimated 21 million Americans with diabetes. Although its incidence and progression can be reduced by intensive glycemic and blood pressure control, nearly all patients with type 1 diabetes mellitus and over 60% of those with type 2 diabetes mellitus eventually develop diabetic retinopathy. There are two stages of diabetic retinopathy. The first, non-proliferative retinopathy, is the earlier stage of the disease and is characterized by increased vascular permeability, microaneurysms, edema and eventually vessel closures. Neovascularization is not a component of the nonproliferative phase. Most visual loss during this stage is due to the fluid accumulating in the macula, the central area of the retina. This accumulation of fluid is called macular edema and can cause temporary or permanent decreased vision. The second stage of diabetic retinopathy is called proliferative retinopathy and is characterized by abnormal new vessel formation. Unfortunately, this abnormal neovascularization can be very damaging because it can cause bleeding in the eye, retinal scar tissue, diabetic retinal detachments or glaucoma, any of which can cause decreased vision or blindness. Macular edema can also occur in the proliferative phase.

Diabetic neuropathy is a common serious complication of diabetes. There are four main types of diabetic neuropathy: peripheral neuropathy, autonomic neuropathy, radiculoplexus neuropathy and mononeuropathy. The signs and symptoms of peripheral neuropathy, the most common type of diabetic neuropathy, include numbness or reduced ability to feel pain or changes in temperature (especially in the feet and toes), a tingling or burning feeling, sharp pain, pain when walking, extreme sensitivity to the lightest touch, muscle weakness, difficulty walking, and serious foot problems (such as ulcers, infections, deformities and bone and joint pain). Autonomic neuropathy affects the autonomic nervous system that controls the heart, bladder, lungs, stomach, intestines, sex organs and eyes, and problems in any of these areas can occur. Radiculoplexus neuropathy (also called diabetic amyotrophy, femoral neuropathy or proximal neuropathy) usually affects nerves in the hips, shoulders or abdomen, usually on one side of the body. Mononeuropathy means damage to just one nerve, typically in an arm, leg or the face. Common complications of diabetic neuropathy include loss of limbs (e.g., toes, feet or legs), charcot joints, urinary tract infections, urinary incontinence, hypoglycemia unawareness (may even be fatal), low blood pressure, digestive problems (*e.g*., constipation, diarrhea, nausea and vomiting), sexual dysfunction (*e.g*., erectile dysfunction), and increased or decreased sweating. As can be seen, symptoms can range from mild to painful, disabling and even fatal.

Diabetic nephropathy is the most common cause of end-stage renal disease in the United States. It is a vascular complication of diabetes that affects the glomerular capillaries of the kidney and reduces the kidney's filtration ability. Nephropathy is first indicated by the appearance of hyperfiltration and then microalbuminuria. Heavy proteinuria and a progressive decline in renal function precede end-stage renal disease. Typically, before any signs of nephropathy appear, retinopathy has usually been diagnosed. Renal transplant is usually recommended to patients with end-stage renal disease due to diabetes. Survival rate at 5 years for patients receiving a transplant is about 60% compared with only 2% for those on dialysis.

Hypertension typically develops over many years, and it affects nearly everyone eventually. Uncontrolled hypertension increases the risk of serious health problems, including heart attack, congestive heart failure, stroke, peripheral artery disease, kidney failure, aneurysms, eye damage, and problems with memory or understanding.

Atherosclerosis also develops gradually. Atherosclerosis can affect the coronary arteries, the carotid artery, the peripheral arteries or the microvasculature, and complications of atherosclerosis include coronary artery disease (which can cause angina or a heart attack), coronary microvascular disease, carotid artery disease (which can cause a transient ischemic attack or stroke), peripheral artery disease (which can cause loss of sensitivity to heat and cold or even tissue death), and aneurysms.

Additional diseases and conditions that can be treated according to the invention include acute lung injury, age-related macular degeneration, choroidal edema, choroiditis, coronary microvascular disease, cerebral microvascular disease, Eales disease, edema caused by injury (*e.g*., trauma or burns), edema associated with hypertension, glomerular vascular leakage, hemorrhagic shock, Irvine Gass Syndrome, edema caused by ischemia, macular edema (*e.g.,* caused by vascular occlusions, post-intraocular surgery *(e.g.,* cataract surgery), uveitis or retinitis pigmentosa, in addition to that caused by diabetes), nephritis (*e.g*., glomerulonephritis, serum sickness nephritis and Thy-1 nephritis), nephropathies, nephrotic edema, nephrotic syndrome, neuropathies, organ failure due to tissue edema (*e.g.,* in sepsis or due to trauma), pre-eclampsia, pulmonary edema, pulmonary hypertension, renal failure, retinal edema, retinal hemorrhage, retinal vein occlusions (*e.g*., branch or central vein occlusions), retinitis, retinopathies (*e.g*., artherosclerotic retinopathy, hypertensive retinopathy, radiation retinopathy, sickle cell retinopathy and retinopathy of prematurity, in addition to diabetic retinopathy), silent cerebral infarction, systemic inflammatory response syndromes (SIRS), transplant glomerulopathy, uveitis, vascular leakage syndrome, vitreous hemorrhage and Von Hippel-Lindau disease. In addition, certain drugs, including those used to treat multiple sclerosis, are known to cause vascular hyperpermeability, and a diketopiperazine, a prodrug of a diketopiperazine or a pharmaceutically-acceptable salt of either one of them, can be used to reduce this unwanted side effect when using these drugs.

"Treat," "treating" or "treatment" is used herein to mean to reduce (wholly or partially) the symptoms, duration or severity of a disease or condition, including curing the disease, or to prevent the disease or condition.

Recent evidence indicates that transcytosis-caused hyperpermeability is the first step of a process that ultimately leads to tissue and organ damage in many diseases and conditions. Accordingly, the present invention provides a means of early intervention in these diseases and conditions which can reduce, delay or even potentially prevent the tissue and organ damage seen in them. For instance, an animal can be treated immediately upon diagnosis of one of the diseases or conditions treatable according to the invention (those diseases and conditions described above).

Alternatively, preferred is the treatment of animals who have early signs of, or a predisposition to develop, such a disease or condition prior to the existence of symptoms. Early signs of, and risk factors for, diabetes, hypertension and atherosclerosis are well known, and treatment of an animal exhibiting these early signs or risk factors can be started prior to the presence of symptoms of the disease or condition (*i.e*., prophylactically).

For instance, treatment of a patient who is diagnosed with diabetes can be started immediately upon diagnosis. In particular, diabetics are to be treated with a diketopiperazine, selected from DA-DKOP, MR-DKP and YE-DKP, or a salt of either of them prior to any symptoms of a vascular complication being present, although this is not usually possible, since most diabetics show such symptoms when they are diagnosed (see below). Alternatively, diabetics should be treated while nonproliferative diabetic retinopathy is mild (*i.e*., mild levels of microaneurysms and intraretinal hemorrhage). See Diabetic Retinopathy, page 9 (Ed. Elia Duh, M.D., Human Press, 2008). Such early treatment will provide the best chance of preventing macular edema and progression of the retinopathy to proliferative diabetic retinopathy. Also, the presence of diabetic retinopathy is considered a sign that other microvascular complications of diabetes exist or will develop (see *Id*., pages 474-477), and early treatment may also prevent or reduce these additional complications. Of course, more advanced diseases and conditions that are vascular complications of diabetes can also be treated with beneficial results.

However, as noted above, vascular complications are often already present by the time diabetes is diagnosed. Accordingly, it is preferable to prophylactically treat a patient who has early signs of, or a predisposition to develop, diabetes. The early signs and risk factors of Type 2 diabetes include fasting glucose that is high, but not high enough to be classified as diabetes ("prediabetes"), hyperinsulinemia, hypertension, dyslipidemia (high cholesterol, high triglycerides, high low-density lipoprotein, and/or low level of high-density lipoprotein), obesity (body mass index above 25), inactivity, over 45 years of age, inadequate sleep, family history of diabetes, minority race, history of gestational diabetes, history of polycystic ovary syndrome and diagnosis of metabolic syndrome. Accordingly, patients with early signs of, or a predisposition to develop, Type 2 diabetes can readily be treated prophylactically.

Similarly, treatment of a patient who is diagnosed with hypertension can be started immediately upon diagnosis. Hypertension typically does not cause any symptoms, but prophylactic treatment can be started in a patient who has a predispostion to develop hypertension. Risk factors for hypertension include age, race (hypertension is more common blacks), family history (hypertension runs in families), overweight or obesity, lack of activity, smoking tobacco, too much salt in the diet, too little potassium in the diet, too little vitamin D in the diet, drinking too much alcohol, high levels of stress, certain chronic conditions (*e.g*., high cholesterol, diabetes, kidney disease and sleep apnea) and use of certain drugs *(e.g.,* oral contraceptives, amphetamines, diet pills, and some cold and allergy medications).

Treatment of a patient who is diagnosed with atherosclerosis can be started immediately upon diagnosis. However, it is preferable to prophylactically treat a patient who has early signs of, or a predispostion to develop, atherosclerosis. Early signs and risk factors for atherosclerosis include age, a family history of aneurysm or early heart disease, hypertension, high cholesterol, high triglycerides, insulin resistance, diabetes, obesity, smoking, lack of physical activity, unhealthy diet, and high level of C-reactive protein.

The method disclosed herein for inhibiting vascular hyperpermeability comprises administering an effective amount of an active ingredient, wherein the active ingredient comprises a diketopiperazine, selected from DA-DKP, MR-DKP and YE-DKP, or a pharmaceutically-acceptable salt of either of them, to an animal in need thereof to inhibit the vascular hyperpermeability.

Methods of making diketopiperazines are well known in the art, and these methods may be employed to synthesize the diketopiperazines of the invention. See, *e.g.,* U.S. Patents Nos. 4,694,081, 5,817,751, 5,990,112, 5,932,579 and 6,555,543, US Patent Application Publication Number 2004/0024180, PCT applications WO 96/00391 and WO 97/48685, and Smith et al., Bioorg. Med. Chem. Letters, 8, 2369-2374 (1998).

For instance, diketopiperazines can be prepared by first synthesizing dipeptides. The dipeptides can be synthesized by methods well known in the art using L-amino acids, D-amino acids or a combination of D- and L-amino acids. Preferred are solid-phase peptide synthetic methods. Of course, dipeptides are also available commercially from numerous sources, including Sigma-Aldrich, St. Louis, MO (primarily custom synthesis), Phoenix Pharmaceuticals, Inc., Belmont, CA (custom synthesis), Fisher Scientific (custom synthesis) and Advanced ChemTech, Louisville, KY.

Once the dipeptide is synthesized or purchased, it is cyclized to form a diketopiperazine. This can be accomplished by a variety of techniques.

For example, U.S. Patent Application Publication Number 2004/0024180 describes a method of cyclizing dipeptides. Briefly, the dipeptide is heated in an organic solvent while removing water by distillation. Preferably, the organic solvent is a low-boiling azeotrope with water, such as acetonitrile, allyl alcohol, benzene, benzyl alcohol, n-butanol, 2-butanol, t-butanol, acetic acid butylester, carbon tetrachloride, chlorobenzene chloroform, cyclohexane, 1,2-dichlorethane, diethylacetal, dimethylacetal, acetic acid ethylester, heptane, methylisobutylketone, 3-pentanol, toluene and xylene. The temperature depends on the reaction speed at which the cyclization takes place and on the type of azeotroping agent used. The reaction is preferably carried out at 50-200°C, more preferably 80-150°C. The pH range in which cyclization takes place can be easily determine by the person skilled in the art. It will advantageously be 2-9, preferably 3-7.

When one or both of the amino acids of the dipeptide has, or is derivatized to have, a carboxyl group on its side chain (e.g., aspartic acid or glutamic acid), the dipeptide is preferably cyclized as described in U.S. Patent No. 6,555,543. Briefly, the dipeptide, with the side-chain carboxyl still protected, is heated under neutral conditions. Typically, the dipeptide will be heated at from about 80°C to about 180°C, preferably at about 120°C. The solvent will be a neutral solvent. For instance, the solvent may comprise an alcohol (such as butanol, methanol, ethanol, and higher alcohols, but not phenol) and an azeotropic co-solvent (such as toluene, benzene, or xylene). Preferably, the alcohol is butan-2-ol, and the azeotropic co-solvent is toluene. The heating is continued until the reaction is complete, and such times can be determined empirically. Typically, the dipeptide will be cyclized by refluxing it for about 8-24 hours, preferably about 18 hours. Finally, the protecting group is removed from the diketopiperazine. In doing so, the use of strong acids (mineral acids, such as sulfuric or hydrochloric acids), strong bases (alkaline bases, such as potassium hydroxide or sodium hydroxide), and strong reducing agents (e.g., lithium aluminum hydride) should be avoided, in order to maintain the chirality of the final compound.

Dipeptides made on solid phase resins can be cyclized and released from the resin in one step. See, *e.g.,* U.S. Patent No. 5,817,751. For instance, the resin having an N-alkylated dipeptide attached is suspended in toluene or toluene/ethanol in the presence of acetic acid *(e.g.,* 1%) or triethylamine *(e.g.,* 4%). Typically, basic cyclization conditions are preferred for their faster cyclization times.

To prepare diketopiperazines wherein the amino acid side chains are derivatized, amino acid derivatives can be used in the synthesis of the dipeptides, the dipeptides can be derivatized and/or the diketopiperazines can be derivatized, as is known in the art. See, *e.g.,* those references cited above.

Other methods of cyclizing dipeptides and of making diketopiperazines are known in the art and can be used in the preparation of diketopiperazines useful in the practice of the invention. See, *e.g.,* those references listed above. In addition, many diketopiperazines can be made from proteins and peptides as described in U.S. Patent No. 7,732,403. Further, diketopiperazines for use in the practice of the invention can be obtained commercially from, *e.g*., Syngene, India or Hemmo Pharmaceuticals Pvt. Ltd., India (both custom synthesis).

The diketopiperazines include all possible stereoisomers that can be obtained by varying the configuration of the individual chiral centers, axes or surfaces. In other words, the diketopierazines include all possible diastereomers, as well as all optical isomers (enantiomers).

"Prodrug" means any compound which releases an active parent drug (a diketopiperazine in this case) *in vivo* when such prodrug is administered to an animal. Prodrugs of diketopiperazines include diketopiperazines derivativatized with any group that may be cleaved *in vivo* to generate the diketopiperazine. Examples of prodrugs include esters.

The physiologically-acceptable salts of the diketopiperazines of the invention may also be used in the practice of the invention. Physiologically-acceptable salts include conventional non-toxic salts, such as salts derived from inorganic acids (such as hydrochloric, hydrobromic, sulfuric, phosphoric, nitric, and the like), organic acids (such as acetic, propionic, succinic, glycolic, stearic, lactic, malic, tartaric, citric, glutamic, aspartic, benzoic, salicylic, oxalic, ascorbic acid, and the like) or bases (such as the hydroxide, carbonate or bicarbonate of a pharmaceutically-acceptable metal cation or organic cations derived from N,N-dibenzylethylenediamine, D-glucosamine, or ethylenediamine). The salts are prepared in a conventional manner, *e.g*., by neutralizing the free base form of the compound with an acid.

As noted above, a diketopiperazine according to the claims or a pharmaceutically-acceptable salt of either one of them can be used to inhibit vascular hyperpermeability and to treat a disease or condition mediated by vascular hyperpermeability. To do so, the diketopiperazine, or pharmaceutically-acceptable salt is administered to an animal in need of treatment. Preferably, the animal is a mammal, such as a rabbit, goat, dog, cat, horse or human. Most preferably, the animal is a human.

A diketopiperazine according to the claims or a pharmaceutically-acceptable salt of either one of them is used in the present invention as an active ingredient. "Active ingredient" is used herein to mean a compound having therapeutic, pharmaceutical or pharmacological activity, and particularly, the therapeutic, pharmaceutical or pharmacological activity described herein. The diketopiperazine, or salt is not used in the present invention as a carrier or as part of a carrier system of a pharmaceutical composition as described in, *e.g.,* U.S. Patents Nos. 5,976,569, 6,099,856, 7,276,534 and PCT applications WO 96/10396, WO 2006/023943, WO 2007/098500, WO 2007/121411 and WO 2010/102148.

Effective dosage forms, modes of administration and dosage amounts for the compounds of the invention (*i.e.,* a diketopiperazine according to the claims or a pharmaceutically-acceptable salt of either one of them) may be determined empirically using the guidance provided herein. It is understood by those skilled in the art that the dosage amount will vary with the particular disease or condition to be treated, the severity of the disease or condition, the route(s) of administration, the duration of the treatment, the identity of any other drugs being administered to the animal, the age, size and species of the animal, and like factors known in the medical and veterinary arts. In general, a suitable daily dose of a compound of the present invention will be that amount of the compound which is the lowest dose effective to produce a therapeutic effect. However, the daily dosage will be determined by an attending physician or veterinarian within the scope of sound medical judgment. If desired, the effective daily dose may be administered as two, three, four, five, six or more sub-doses, administered separately at appropriate intervals throughout the day. Administration of the compound should be continued until an acceptable response is achieved.

In particular, an effective dosage amount of a compound of the invention for inhibiting vascular hyperpermeability will be from 10 ng/kg/day to 225 mg/kg/day, preferably from 500 ng/kg/day to 150 mg/kg/day, most preferably from 1 mg/kg/day to 30 mg/kg/day. When given orally to an adult human, the dose will preferably be from about 1 mg/day to about 10 g/day, more preferably the dose will be from about 60 mg/day to about 6 g/day, most preferably the dose will be from about 100 mg/day to about 1200 mg/day, preferably given in several doses.

The present disclosure also relates to a method of modulating the cytoskeleton of endothelial cells in an animal. Modulation of the cytoskeleton can reduce vascular hyperpermeability and increase vascular hypopermeability (*i.e*., permeability below basal levels), thereby returning the endothelium to homeostasis. Accordingly, those diseases and conditions mediated by vascular hyperpermeability can be treated (see above) and those diseases and conditions mediated by vascular hypopermeability can also be treated. The latter type of diseases and conditions include aging liver, atherogenesis, atherosclerosis, cirrhosis, fibrosis of the liver, liver failure and primary and metastatic liver cancers.

The method of modulating the cytoskeleton of endothelial cells comprises administering an effective amount of a diketopiperazine according to the claims or a pharmaceutically-acceptable salt of either one of them, to the animal. The diketopiperazines are the same as those described above for inhibiting vascular hyperpermeability, and "animal" has the same meaning as set forth above.

Effective dosage forms, modes of administration and dosage amounts for the compounds of the invention for modulating the cytoskeleton may be determined empirically using the guidance provided herein. It is understood by those skilled in the art that the dosage amount will vary with the particular disease or condition to be treated, the severity of the disease or condition, the route(s) of administration, the duration of the treatment, the identity of any other drugs being administered to the animal, the age, size and species of the animal, and like factors known in the medical and veterinary arts. In general, a suitable daily dose of a compound of the present invention will be that amount of the compound which is the lowest dose effective to produce a therapeutic effect. However, the daily dosage will be determined by an attending physician or veterinarian within the scope of sound medical judgment. If desired, the effective daily dose may be administered as two, three, four, five, six or more sub-doses, administered separately at appropriate intervals throughout the day. Administration of the compound should be continued until an acceptable response is achieved.

In particular, an effective dosage amount of a compound of the invention for modulating the cytoskeleton of endothelial cells will be from 10 ng/kg/day to 225 mg/kg/day, preferably from 500 ng/kg/day to 150 mg/kg/day, most preferably from 1 mg/kg/day to 30 mg/kg/day. When given orally to an adult human, the dose will preferably be from about 1 mg/day to about 10000 mg/day, more preferably the dose will be from about 60 mg/day to about 6000 mg/day, most preferably the dose will be from about 100 mg/day to about 1200 mg/day, preferably given in several doses.

The compounds of the present invention may be administered to an animal patient for therapy by any suitable route of administration, including orally, nasally, parenterally (e.g., intravenously, intraperitoneally, subcutaneously or intramuscularly), transdermally, intraocularly and topically (including buccally and sublingually). Generally preferred is oral administration for any disease or condition treatable according to the invention. The preferred routes of administration for treatment of diseases and conditions of the eye are orally, intraocularly and topically. Most preferred is orally. The preferred routes of administration for treatment of diseases and conditions of the brain are orally and parenterally. Most preferred is orally.

While it is possible for a compound of the present invention to be administered alone, it is preferable to administer the compound as a pharmaceutical formulation (composition). The pharmaceutical compositions of the invention comprise a compound or compounds of the invention as an active ingredient in admixture with one or more pharmaceutically-acceptable carriers and, optionally, with one or more other compounds, drugs or other materials. Each carrier must be "acceptable" in the sense of being compatible with the other ingredients of the formulation and not injurious to the animal. Pharmaceutically-acceptable carriers are well known in the art. Regardless of the route of administration selected, the compounds of the present invention are formulated into pharmaceutically-acceptable dosage forms by conventional methods known to those of skill in the art. See, *e.g*., *Remington's Pharmaceutical Sciences.*

Formulations of the invention suitable for oral administration may be in the form of capsules, cachets, pills, tablets, powders, granules or as a solution or a suspension in an aqueous or non-aqueous liquid, or an oil-in-water or water-in-oil liquid emulsions, or as an elixir or syrup, or as pastilles (using an inert base, such as gelatin and glycerin, or sucrose and acacia), and the like, each containing a predetermined amount of a compound or compounds of the present invention as an active ingredient. A compound or compounds of the present invention may also be administered as bolus, electuary or paste.

In solid dosage forms of the invention for oral administration (capsules, tablets, pills, dragees, powders, granules and the like), the active ingredient (*i.e*., a diketopiperazine according to the claims pharmaceutically-acceptable salt of either one of them, or combinations of the foregoing) is mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monosterate; (8) absorbents, such as kaolin and bentonite clay; (9) lubricants, such as talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may be employed as fillers in soft and hard-filled gelatin capsules using such excipients as lactose or milk sugars, as well as high molecular weight polyethylene glycols and the like.

A tablet may be made by compression or molding optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter. These compositions may also optionally contain opacifying agents and may be of a composition that they release the active ingredient only, or preferentially, in a certain portion of the gastrointestinal tract, optionally, in a delayed manner. Examples of embedding compositions which can be used include polymeric substances and waxes. The active ingredient can also be in microencapsulated form.

Liquid dosage forms for oral administration of the compounds of the invention include pharmaceutically-acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active ingredient, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

The invention also provides pharmaceutical products suitable for treatment of the eye. Such pharmaceutical products include pharmaceutical compositions, devices and implants (which may be compositions or devices).

Pharmaceutical formulations (compositions) for intraocular injection of a compound or compounds of the invention into the eyeball include solutions, emulsions, suspensions, particles, capsules, microspheres, liposomes, matrices, etc. See, *e.g.,* U.S. Patent No. 6,060,463, U.S. Patent Application Publication No. 2005/0101582, and PCT application WO 2004/043480. For instance, a pharmaceutical formulation for intraocular injection may comprise one or more compounds of the invention in combination with one or more pharmaceutically-acceptable sterile isotonic aqueous or non-aqueous solutions, suspensions or emulsions, which may contain antioxidants, buffers, suspending agents, thickening agents or viscosity-enhancing agents (such as a hyaluronic acid polymer). Examples of suitable aqueous and nonaqueous carriers include water, saline (preferably 0.9%), dextrose in water (preferably 5%), buffers, dimethylsulfoxide, alcohols and polyols (such as glycerol, propylene glycol, polyethylene glycol, and the like). These compositions may also contain adjuvants such as wetting agents and emulsifying agents and dispersing agents. In addition, prolonged absorption of the injectable pharmaceutical form may be brought about by the inclusion of agents which delay absorption such as polymers and gelatin. Injectable depot forms can be made by incorporating the drug into microcapsules or microspheres made of biodegradable polymers such as polylactide-polyglycolide. Examples of other biodegradable polymers include poly(orthoesters), poly(glycolic) acid, poly(lactic) acid, polycaprolactone and poly(anhydrides). Depot injectable formulations are also prepared by entrapping the drug in liposomes (composed of the usual ingredients, such as dipalmitoyl phosphatidylcholine) or microemulsions which are compatible with eye tissue. Depending on the ratio of drug to polymer or lipid, the nature of the particular polymer or lipid components, the type of liposome employed, and whether the microcapsules or microspheres are coated or uncoated, the rate of drug release from microcapsules, microspheres and liposomes can be controlled.

The compounds of the invention can also be administered surgically as an ocular implant. For instance, a reservoir container having a diffusible wall of polyvinyl alcohol or polyvinyl acetate and containing a compound or compounds of the invention can be implanted in or on the sclera. As another example, a compound or compounds of the invention can be incorporated into a polymeric matrix made of a polymer, such as polycaprolactone, poly(glycolic) acid, poly(lactic) acid, poly(anhydride), or a lipid, such as sebacic acid, and may be implanted on the sclera or in the eye. This is usually accomplished with the animal receiving a topical or local anesthetic and using a small incision made behind the cornea. The matrix is then inserted through the incision and sutured to the sclera.

The compounds of the invention can also be administered topically to the eye, and a preferred embodiment of the invention is a topical pharmaceutical composition suitable for application to the eye. Topical pharmaceutical compositions suitable for application to the eye include solutions, suspensions, dispersions, drops, gels, hydrogels and ointments. See, *e.g*., U.S. Patent No. 5,407,926 and PCT applications WO 2004/058289, WO 01/30337 and WO 01/68053.

Topical formulations suitable for application to the eye comprise one or more compounds of the invention in an aqueous or nonaqueous base. The topical formulations can also include absorption enhancers, permeation enhancers, thickening agents, viscosity enhancers, agents for adjusting and/or maintaining the pH, agents to adjust the osmotic pressure, preservatives, surfactants, buffers, salts (preferably sodium chloride), suspending agents, dispersing agents, solubilizing agents, stabilizers and/or tonicity agents. Topical formulations suitable for application to the eye will preferably comprise an absorption or permeation enhancer to promote absorption or permeation of the compound or compounds of the invention into the eye and/or a thickening agent or viscosity enhancer that is capable of increasing the residence time of a compound or compounds of the invention in the eye. See PCT applications WO 2004/058289, WO 01/30337 and WO 01/68053. Exemplary absorption/permeation enhancers include methysulfonylmethane, alone or in combination with dimethylsulfoxide, carboxylic acids and surfactants. Exemplary thickening agents and viscosity enhancers include dextrans, polyethylene glycols, polyvinylpyrrolidone, polysaccharide gels, Gelrite®, cellulosic polymers (such as hydroxypropyl methylcellulose), carboxyl-containing polymers (such as polymers or copolymers of acrylic acid), polyvinyl alcohol and hyaluronic acid or a salt thereof.

Liquid dosage forms (e.g., solutions, suspensions, dispersions and drops) suitable for treatment of the eye can be prepared, for example, by dissolving, dispersing, suspending, etc. a compound or compounds of the invention in a vehicle, such as, for example, water, saline, aqueous dextrose, glycerol, ethanol and the like, to form a solution, dispersion or suspension. If desired, the pharmaceutical formulation may also contain minor amounts of non-toxic auxillary substances, such as wetting or emulsifying agents, pH buffering agents and the like, for example sodium acetate, sorbitan monolaurate, triethanolamine sodium acetate, triethanolamine oleate, etc.

Aqueous solutions and suspensions suitable for treatment of the eye can include, in addition to a compound or compounds of the invention, preservatives, surfactants, buffers, salts (preferably sodium chloride), tonicity agents and water. If suspensions are used, the particle sizes should be less than 10 µm to minimize eye irritation. If solutions or suspensions are used, the amount delivered to the eye should not exceed 50 µl to avoid excessive spillage from the eye.

Colloidal suspensions suitable for treatment of the eye are generally formed from microparticles (*i.e*., microspheres, nanospheres, microcapsules or nanocapsules, where microspheres and nanospheres are generally monolithic particles of a polymer matrix in which the formulation is trapped, adsorbed, or otherwise contained, while with microcapsules and nanocapsules the formulation is actually encapsulated). The upper limit for the size of these microparticles is about 5µ to about 10µ.

Ophthalmic ointments suitable for treatment of the eye include a compound or compounds of the invention in an appropriate base, such as mineral oil, liquid lanolin, white petrolatum, a combination of two or all three of the foregoing, or polyethylene-mineral oil gel. A preservative may optionally be included.

Ophthalmic gels suitable for treatment of the eye include a compound or compounds of the invention suspended in a hydrophilic base, such as Carpobol-940 or a combination of ethanol, water and propylene glycol *(e.g.,* in a ratio of 40:40:20). A gelling agent, such as hydroxylethylcellulose, hydroxypropylcellulose, hydroxypropylmethylcellulose or ammoniated glycyrrhizinate, is used. A preservative and/or a tonicity agent may optionally be included.

Hydrogels suitable for treatment of the eye are formed by incorporation of a swellable, gel-forming polymer, such as those listed above as thickening agents or viscosity enhancers, except that a formulation referred to in the art as a "hydrogel" typically has a higher viscosity than a formulation referred to as a "thickened" solution or suspension. In contrast to such preformed hydrogels, a formulation may also be prepared so to form a hydrogel *in situ* following application to the eye. Such gels are liquid at room temperature but gel at higher temperatures (and thus are termed "thermoreversible" hydrogels), such as when placed in contact with body fluids. Biocompatible polymers that impart this property include acrylic acid polymers and copolymers, N-isopropylacrylamide derivatives and ABA block copolymers of ethylene oxide and propylene oxide (conventionally referred to as "poloxamers" and available under the Pluronic® tradename from BASF-Wayndotte).

Preferred dispersions are liposomal, in which case the formulation is enclosed within liposomes (microscopic vesicles composed of alternating aqueous compartments and lipid bilayers).

Eye drops can be formulated with an aqueous or nonaqueous base also comprising one or more dispersing agents, solubilizing agents or suspending agents. Drops can be delivered by means of a simple eye dropper-capped bottle or by means of a plastic bottle adapted to deliver liquid contents dropwise by means of a specially shaped closure.

The compounds of the invention can also be applied topically by means of drug-impregnated solid carrier that is inserted into the eye. Drug release is generally effected by dissolution or bioerosion of the polymer, osmosis, or combinations thereof. Several matrix-type delivery systems can be used. Such systems include hydrophilic soft contact lenses impregnated or soaked with the desired compound of the invention, as well as biodegradable or soluble devices that need not be removed after placement in the eye. These soluble ocular inserts can be composed of any degradable substance that can be tolerated by the eye and that is compatible with the compound of the invention that is to be administered. Such substances include, but are not limited to, poly(vinyl alcohol), polymers and copolymers of polyacrylamide, ethylacrylate and vinylpyrrolidone, as well as cross-linked polypeptides or polysaccharides, such as chitin.

Dosage forms for the other types of topical administration (*i.e*., not to the eye) or for transdermal administration of compounds of the invention include powders, sprays, ointments, pastes, creams, lotions, gels, solutions, patches, drops and inhalants. The active ingredient may be mixed under sterile conditions with a pharmaceutically-acceptable carrier, and with any buffers, or propellants which may be required. The ointments, pastes, creams and gels may contain, in addition to the active ingredient, excipients, such as animal and vegetable fats, oils, waxes, paraffins, starch, tragacanth, cellulose derivatives, polyethylene glycols, silicones, bentonites, silicic acid, talc and zinc oxide, or mixtures thereof. Powders and sprays can contain, in addition to the active ingredient, excipients such as lactose, talc, silicic acid, aluminum hydroxide, calcium silicates and polyamide powder or mixtures of these substances. Sprays can additionally contain customary propellants such as chlorofluorohydrocarbons and volatile unsubstituted hydrocarbons, such as butane and propane. Transdermal patches have the added advantage of providing controlled delivery of compounds of the invention to the body. Such dosage forms can be made by dissolving, dispersing or otherwise incorporating one or more compounds of the invention in a proper medium, such as an elastomeric matrix material. Absorption enhancers can also be used to increase the flux of the compound across the skin. The rate of such flux can be controlled by either providing a rate-controlling membrane or dispersing the compound in a polymer matrix or gel. A drug-impregnated solid carrier *(e.g.,* a dressing) can also be used for topical administration.

Pharmaceutical formulations include those suitable for administration by inhalation or insufflation or for nasal administration. For administration to the upper (nasal) or lower respiratory tract by inhalation, the compounds of the invention are conveniently delivered from an insufflator, nebulizer or a pressurized pack or other convenient means of delivering an aerosol spray. Pressurized packs may comprise a suitable propellant such as dichlorodifluoromethane, trichlorofluoromethane, dichlorotetrafluoroethane, carbon dioxide, or other suitable gas. In the case of a pressurized aerosol, the dosage unit may be determined by providing a valve to deliver a metered amount.

Alternatively, for administration by inhalation or insufflation, the composition may take the form of a dry powder, for example, a powder mix of one or more compounds of the invention and a suitable powder base, such as lactose or starch. The powder composition may be presented in unit dosage form in, for example, capsules or cartridges, or, *e.g.,* gelatin or blister packs from which the powder may be administered with the aid of an inhalator, insufflator or a metered-dose inhaler.

For intranasal administration, compounds of the invention may be administered by means of nose drops or a liquid spray, such as by means of a plastic bottle atomizer or metered-dose inhaler. Liquid sprays are conveniently delivered from pressurized packs. Typical of atomizers are the Mistometer (Wintrop) and Medihaler (Riker).

Nose drops may be formulated with an aqueous or nonaqueous base also comprising one or more dispersing agents, solubilizing agents or suspending agents. Drops can be delivered by means of a simple eye dropper-capped bottle or by means of a plastic bottle adapted to deliver liquid contents dropwise by means of a specially shaped closure.

Pharmaceutical compositions of this invention suitable for parenteral administrations comprise one or more compounds of the invention in combination with one or more pharmaceutically-acceptable sterile isotonic aqueous or non-aqueous solutions, dispersions, suspensions or emulsions, or sterile powders which may be reconstituted into sterile injectable solutions or dispersions just prior to use, which may contain antioxidants, buffers, solutes which render the formulation isotonic with the blood of the intended recipient or suspending or thickening agents. Also, drug-coated stents may be used.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as wetting agents, emulsifying agents and dispersing agents. It may also be desirable to include isotonic agents, such as sugars, sodium chloride, and the like in the compositions. In addition, prolonged absorption of the injectable pharmaceutical form may be brought about by the inclusion of agents which delay absorption such as aluminum monosterate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug is accomplished by dissolving or suspending the drug in an oil vehicle.

Injectable depot forms are made by forming microencapsule matrices of the drug in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissue. The injectable materials can be sterilized for example, by filtration through a bacterial-retaining filter.

The formulations may be presented in unit-dose or multi-dose sealed containers, for example, ampules and vials, and may be stored in a lyophilized condition requiring only the addition of the sterile liquid carrier, for example water for injection, immediately prior to use. Extemporaneous injection solutions and suspensions may be prepared from sterile powders, granules and tablets of the type described above.

The diketopiperazine according to the claims or a pharmaceutically-acceptable salt of either one of them, may be given alone to treat a disease or condition involving vascular hyperpermeability or dysfunction of the cytoskeleton. Alternatively, the diketopiperazine, or salt may be given in combination with each other and/or in combination with one or more other treatments or drugs suitable for treating the disease or condition. For instance, the diketopiperazine, or the salt can be administered prior to, in conjunction with (including simultaneously with), or after the other treatment or drug. In the case of another drug, the drug and the diketopiperazine, or salt, may be administered in separate pharmaceutical compositions or as part of the same pharmaceutical composition.

The invention also provides kits. The kits comprise a container holding a diketopiperazine according to the claims or a pharmaceutically-acceptable salt of either of them. The kits may further comprise one or more additional containers each holding one or more other drugs suitable for use in the methods of the invention. Suitable containers include vials, bottles (including with a bottle with a dropper or a squeeze bottle), blister packs, inhalers, jars, nebulizers, packets (e.g., made of foil, plastic, paper, cellophane or another material), syringes and tubes. The kit will also contain instructions for administration of the diketopiperazine, prodrug or salt and, optionally, the one or more other drugs suitable for use in the methods of the invention. The instructions may, for instance, be printed on the packaging holding the container(s), may be printed on a label attached to the kit or the container(s), or may be printed on a separate sheet of paper that is included in or with the kit. The packaging holding the container(s) may be, for instance, a box, or the container(s) may wrapped in, for instance, plastic shrink wrap. The kit may also contain other materials which are known in the art and which may be desirable from a commercial and user standpoint. For instance, the kit may contain instructions to help a patient manage his/her diabetes or hypertension.

As used herein, "a" or "an" means one or more.

As used herein, "comprises" and "comprising" include within their scope all narrower terms, such as "consisting essentially of" and "consisting of" as alternative embodiments of the present invention characterized herein by "comprises" or "comprising". In regard to use of "consisting essentially of", this phrase limits the scope of a claim to the specified steps and materials and those that do not materially affect the basic and novel characteristics of the invention disclosed herein. The basic and novel characteristics of the invention can be inhibition of vascular hyperpermeability, modulation of a cytoskeleton of an endothelial cell, or both, in an animal.

Additional objects, advantages and novel features of the present invention will become apparent to those skilled in the art by consideration of the following non-limiting examples.

### EXAMPLES

### Example 1: Effect of DA-DKP on ECIS

Assays were performed to determine the effect of DA-DKP on transendothelial electrical resistance (TER) of human renal glomerular microvascular endothelial cells (ACBRI 128, Cell Systems Corporation (exclusive distributor for Applied Cell Biology Research Institute), Kirkland, WA). Electrical resistance was measured using the electric cell-substrate impedance sensing (ECIS) system (ECISZθ, obtained from Applied Biophysics) with 8-well multiple electrode plates (8W10E). Each well of the plates was coated with 5 µg/cm² fibronectin in HBSS by adding the fibronectin in a volume of 100 µl per well and incubating the plates for 30 minutes in a 37°C incubator with 5% CO₂. The fibronectin solution was removed, and 400 µl of EGM-2 culture medium (Lonza) was added to each well. The plates were connected to the ECISZθ system and were electrically stabilized. The EGM-2 medium was aspirated and replaced with 200 µl of EGM-2 culture medium containing 100,000 cells per well. The plates were reconnected to the ECISZθ system and incubated for 24 hours in a 37°C incubator with 5% CO₂. The EGM-2 medium was aspirated and replaced with 400 µl of fresh EGM-2 culture medium per well. The plates were reconnected to the ECISZθ system and incubated for 6 hours in a 37°C incubator with 5% CO₂. Solutions of the test compounds in HBSS were prepared and placed in the incubator to equilibrate. The test compounds were then added to appropriate wells at the following final concentrations: DA-DKP (100 µM) (Sigma) and TNFα (1 ng/ml) (Sigma). ECIS (resistance) was monitored for 90 hours.

In the glomerular endothelial cells, 100 µM DA-DKP alone showed an increase of ECIS as compared to untreated cells starting about 5.0 hours, reaching significance at 12 hours, and persisting for 35 hours, after treatment. While not significant, DA-DKP showed an ability to prevent some of the TNFα-induced drop in ECIS.

### Example 2: Effect of DA-DKP on ECIS

Assays were performed to determine the effect of DA-DKP on transendothelial electrical resistance (TER) of human retinal endothelial cells (ACBRI 181, Cell Systems Corporation (exclusive distributor for Applied Cell Biology Research Institute), Kirkland, WA). Electrical resistance was measured using the electric cell-substrate impedance sensing (ECIS) system (ECISZθ, obtained from Applied Biophysics) as described in Example 1, but using 96-well multiple electrode plates (8W10E). Also, several does of DA-DKP were used (0.5 µM, 5.0 µM, 50 µM and 100 µM). DA-DKP gave a dose-dependent increase in ECIS (TER), with 100 µM giving the greatest increase.

### Example 3: Effect of DA-DKP on Actin Stress Fiber Formation

Passage 12 human retinal endothelial cells (ACBRI 181, Cell Systems Corporation (exclusive distributor for Applied Cell Biology Research Institute), Kirkland, WA) were seeded into 16-chamber glass slides coated with 5 µg/cm² fibronectin at 5000 cells per well in a total volume of 200 µl of EGM-2 medium (Lonza). The slides were cultured in a 37°C incubator with 5% CO₂ for 48 hours with daily medium changes. Then, the test compounds (DA-DKP, S1P and TNFα), diluted in Hanks Balanced Salt Solution (HBSS; Lonza), were added to give the following final concentrations: DA-DKP (100 µM) (Sigma), TNFα (1 ng/ml) (Sigma), and S1P (1 µM) (Sigma). The slides were incubated with the test compounds for 15 minutes or 3 hours in a 37°C incubator with 5% CO₂. After this incubation, the medium was aspirated, and the cells were fixed using 3.6% formaldehyde in phosphate buffered saline (PBS) for ten minutes at room temperature. All wells were then washed two times with 100 µl PBS. The cells were permeabilized using a 0.1% Triton X-100 in PBS for 5 minutes. All wells were then washed two times with 100 µl PBS, and 50 µl of a 1:40 dilution of rhodamine-phalloidin (Invitrogen) in PBS was added to the cells to stain for F-actin and left on the cells for 20 minutes at room temperature. All wells were then washed two times with 100 µl PBS. Then 100 µl PBS was added to each well and the cells were observed and photographed using an inverted microscope using a rhodamine (ex530/em590) filter.

The retinal endothelial cells treated with DA-DKP alone showed diffuse membrane f-actin staining at 15 minute and at 3 hours. With TNFα alone, stress fibers were seen at all times, with the number of cells exhibiting stress fibers and the thickness of the fibers increasing from 15 minutes to 3 hours. DA-DKP decreased the stress fiber formation and/or the thickness of the fibers caused by TNFα at both times. Cells treated with S1P alone showed actin cortical rings, at 15 minutes and 3 hours. DA-DKP seemed to enhance the cortical rings at 15 minutes and 3 hours.

S1P (sphingosine-1 phosphate) plays a very important function in the formation and maintenance of vascular endothelium. S1P is a constitutive signaling input that facilitates the organization and barrier function of the vascular endothelium through its effects on the actin cytoskeletion. In particular, S1P is involved in the formation of cortical actin fibers and organization of the adherens junctions. Depletion of S1P leads to vascular leak and edema, and S1P can reverse endothelial dysfunction and restore barrier function.

In this experiment, DA-DKP exhibited an ability to strengthen the protective effects of S1P in retinal endothelial cells. DA-DKP also reversed the formation of stress fibers induced by TNFα. Diffuse perinuclear staining is seen in cells treated with DA-DKP alone.

### Example 4: Effect of DA-DKP on RhoA

Remodeling of the endothelial cell cytoskeleton is central to many functions of the endothelium. The Rho family of small GTP-binding proteins have been identified as key regulators of F-actin cytoskeletal dynamics. The Rho family consists of three isoforms, RhoA, RhoB and RhoC. The activation of RhoA activity leads to prominent stress fiber formation in endothelial cells. Stimulation of endothelial cells with thrombin increases Rho GTP and myosin phosphorylation, consistent with increased cell contractility. Inhibition of RhoA blocks this response and the loss of barrier function, demonstrating a critical role for Rho in vascular permeability.

This experiment was performed using a commercially-available Rho activation assay (GLISA) purchased from Cytoskeleton, Denver, Colorado, following the manufacturer's protocol. Briefly, passage 8 or 12 human retinal endothelial cells (ACBRI 181, Applied Cell Biology Research Institute, Kirkland, WA) were cultured on fibronectin-coated (1 µg/cm²) 6-well tissue culture plates using EGM-2 culture medium (Lonza) for 24 hours in a 37°C incubator with 5% CO₂ (30,000 cells /well in total volume of 3 ml). Then, the medium was aspirated and replaced with Ultraculture medium supplemented with 0.1% fetal bovine serum, L-glutamine, sodium pyruvate, penicillin/streptomycin and ITSS (insulin, transferrin sodium selenium) (all from Lonza) to serum starve the cells and reduce the background level of RhoA. The cells were cultured for 24 hours in a 37°C incubator with 5% CO₂. Test compounds diluted in HBSS were placed in the incubator to equilibrate before addition to the cells. Then, 150 µl of each test compound was added to the appropriate culture wells, and the plates were incubated in the incubator for an additional 15 minutes. Then, thrombin was added to appropriate wells. After 1 minute, the cells were washed one time with 1.5 ml phosphate buffered saline and were then lysed with 100 µl GLISA lysis buffer supplemented with protease inhibitors. The extracts were scraped, transferred to microcentrifuge tubes and transferred to ice to preserve the active form of RhoA. All extracts were then cleared of debris by spinning at 10,000 rpms for 2 minutes at 4°C. The supernatants were transferred to new tubes and placed back on ice. Aliquots of each extract were removed for the GLISA assay and for protein determinations. All protein concentrations were within 10%, and the extracts were used at the achieved concentrations (equates to 15 µg total protein per well). The GLISA assay was performed using the reagents supplied in the kit.

The results for the passage 12 retinal endothelial cells are presented in Table 1 below. As expected, the active Rho A levels induced by thrombin were very high. All of the test compounds inhibited the thrombin-induced activation of Rho A.

The results for the passage 8 retinal endothelial cells are presented in Table 2 below. As expected, the active Rho A levels induced by thrombin were very high. All of the test compounds inhibited the thrombin-induced activation of Rho A.

**TABLE 1**

| Treatment | Mean OD | Percent Inhibition vs. Untreated Control | Percent Inhibition vs. Thrombin |
|---|---|---|---|
| Untreated | 0.455 | --- | --- |
| 100 µM DA-DKP | 0.389 | 14.52 | --- |
| 1.0 µM Dexamethasone | 0.428 | 5.83 | --- |
| 10.0 µM PI3 kinase inhibitor LY 294002 | 0.370 | 18.70 | --- |
| 1.0 µM Src-1 Inhibitor* | 0.349 | 23.21 | --- |
| 0.1 U/ml Thrombin | 1.013 | --- | --- |
| 0.1 U/ml Thrombin + 100 µM DA-DKP | 0.752 | --- | 46.82 |
| 0.1 U/ml Thrombin + 1.0 µM Dexamethasone | 0.826 | --- | 33.48 |
| 0.1 U/ml Thrombin + 10.0 µM PI3 kinase inhibitor LY294002 | 0.685 | --- | 58.73 |
| 0.1 U/ml Thrombin + 1.0 µM Src-1 Inhibitor | 0.534 | --- | 85.85 |

| | | | |
|---|---|---|---|
| * Obtained from Sigma. | | | |

**TABLE 2**

| Treatment | Mean OD | Percent Inhibition vs. Untreated Control | Percent Inhibition vs. Thrombin |
|---|---|---|---|
| Untreated | 0.102 | --- | --- |
| 100 µM DA-DKP | 0.110 | -7.88 | --- |
| 10.0 µM PI3 kinase inhibitor LY 294002 | 0.056 | 45.32 | --- |
| 0.1 U/ml Thrombin | 0.561 | --- | --- |
| 0.1 U/ml Thrombin + 100 µM DA-DKP | 0.377 | --- | 40.04 |
| 0.1 U/ml Thrombin + 10.0 µM PI3 kinase inhibitor LY294002 | 0.433 | --- | 27.86 |

### Example 5: Effect of MR-DKP on ECIS

Assays were performed to determine the effect of MR-DKP (a diketopiperazine wherein R¹ in formula I is the side chain of methionine and R² is side chain of arginine) on transendothelial electrical resistance (TER) of passage 6 human retinal endothelial cells (Appplied Cell Systems Corporation (exclusive distributor for Applied Cell Biology Research Institute), Kirkland, WA). Electrical resistance was measured using the electric cell-substrate impedance sensing (ECIS) system (ECISZθ, obtained from Applied Biophysics) with 8-well multiple electrode plates (8W10E). Each well of the plates was stabilized by adding 250 µl of 10 mM cysteine (Sigma) in sterile water to each well and incubating for 30 minutes at room temperature. The wells were then washed two times with 150 µl of sterile water to remove the cysteine. All wells were then coated with 10 µg/cm² collagen by diluting the stock solution (0.5 mg/ml Type IV collagenin 0.25% acetic acid (Sigma) in sterile water and adding 150 µl of the resulting solution to each well. The collagen solution was incubated on the plates at 37°C for 120 minutes and then removed. The wells were washed two times with 400 µl sterile water to remove the collagen. Next, 400 µl of EGM-2 culture medium (Lonza) was added to each well. The plates were connected to the ECISZθ system and were electrically stabilized. The EGM-2 medium was aspirated and replaced with 400 µl of EGM-2 culture medium containing 100,000 cells per well. The plates were reconnected to the ECISZθ system and incubated for 24 hours in a 37°C incubator with 5% CO₂. The EGM-2 medium was aspirated and replaced with 400 µl of EGM-2 culture medium. The plates were reconnected to the ECISZθ system and incubated for 2 hours in a 37°C incubator with 5% CO₂. Solutions of the test compound in HBSS were prepared and placed in the incubator to equilibrate. The test compound was then added to appropriate wells at the following final concentrations: MR-DKP (50µM and 100 µM). ECIS (resistance) was monitored for 50 hours.

In the retinal endothelial cells, both 50 µM and 100 µM MR-DKP showed an increase in ECIS as compared to untreated cells starting at about 15 hours, becoming significant at about 18 hours. The increase was around 20% at its maximum. For the 100 µM group, the increase persisted for the remainder of the experiment, reaching significance again at about 33 hours. For the 50 µM group, at around 28-29 hours, the resistance returned to the levels of the control, but increased again beginning at about 30 hours, reaching significance at about 33 hours, and the increase persisted for the remainder of the experiment. In addition, the 50 µM group showed a brief elevation in resistance from 2-5 hours.

### Example 6: Effect of YE-DKP on ECIS

Example 5 was repeated, except that the diketopiperazine used was YE-DKP (a diketopiperazine wherein R¹ in formula I is the side chain of glutamic acid and R² is the side chain of tyrosine). In the retinal endothelial cells, 50 µM YE-DKP did not show a significant increase in ECIS, but 100 µM YE-DKP showed an increase in ECIS as compared to untreated cells starting at about 6 hours, becoming significant at about 12 hours. The increase was about 20% at its maximum. At around 28 hours, the resistance returned to the levels of the control, but increased again beginning at about 29 hours, reaching significance at about 33 hours, and the increase persisted for the remainder of the experiment.

## Claims

1. An active ingredient comprising a diketopiperazine, or a pharmaceutically-acceptable salt thereof, for use in inhibiting vascular hyperpermeability in an animal having a disease or condition mediated by vascular hyperpermeability selected from the group consisting diabetic macular edema, age-related macular degeneration, myocardial edema, myocardial fibrosis, diastolic dysfunction, diabetic cardiomyopathy, retardation of lung development in the fetuses of diabetic mothers, vascular hyperplasy in the mesentery, diabetic neuropathy, diabetic nephropathy, diabetic retinopathy, acute lung injury, acute respiratory distress syndrome, atherosclerosis, choroidal edema, choroiditis, coronary microvascular disease, cerebral microvascular disease, Eales disease, edema caused by injury, edema associated with hypertension, hemorrhagic shock, hypertension, Irvine Gass Syndrome, ischemia, macular edema, nephritis, nephropathies, nephrotic edema, nephrotic syndrome, neuropathy, organ failure due to edema, pre-eclampsia, pulmonary edema, pulmonary hypertension, renal failure, retinal edema, retinal hemorrhage, retinal vein occlusion, retinitis, retinopathy, silent cerebral infarction, systemic inflammatory response syndrome, transplant glomerulopathy, vascular leakage syndrome, vitreous hemorrhage and Von Hippel-Lindau disease, comprising administering to the animal an effective amount of the active ingredient, wherein the diketopiperazine is selected from the group consisting of DA-DKP, MR-DKP and YE-DKP.

2. The active ingredient for use according to claim 1 wherein administration of the diketopiperazine or salt is commenced immediately upon diagnosis of the disease or condition.

3. The active ingredient for use according to claim 1 wherein the animal is in need of the diketopiperazine or salt because of one or more early signs of, or a predisposition to develop, a disease or condition mediated by vascular hyperpermeability.

4. The active ingredient for use according to claim 3 wherein the disease or condition is diabetic macular edema or age-related macular degeneration.

5. The active ingredient for use according to claim 1 wherein the vascular hyperpermeability is vascular hyperpermeability of a continuous endothelium found in, or
around, a brain, diaphragm, duodenal musculature, fat, heart, kidney, large blood vessel, lung, mesentery, nerve, retina, skeletal muscle, skin or testis.

6. The active ingredient for use according to claim 1 wherein the vascular hyperpermeability is vascular hyperpermeability of a fenestrated endothelium found in, or around, a kidney, a pancreas, an adrenal, an endocrine gland or an intestine.

7. The active ingredient for use according to any one of claims 1 to 6 wherein the diketopiperazine or salt is administered orally.

8. The active ingredient for use according to any one of claims 1 to 7 wherein the animal is a human.

## Patentansprüche

1. Ein Wirkstoff, der ein Diketopiperazin oder ein pharmazeutisch verträgliches Salz davon beinhaltet, zur Verwendung bei der Hemmung der vaskulären Hyperpermeabilität in einem Tier, das eine Erkrankung oder einen Krankheitszustand aufweist, die oder der durch vaskuläre Hyperpermeabilität vermittelt wird, ausgewählt aus der Gruppe, die aus Folgenden besteht: diabetischem Makulaödem, altersbedingter Makuladegeneration, Myokardödem, Myokardfibrose, diastolischer Dysfunktion, diabetischer Kardiomyopathie, Verzögerung der Lungenentwicklung bei Feten diabetischer Mütter, vaskulärer Hyperplasie im Mesenterium, diabetischer Neuropathie, diabetischer Nephropathie, diabetischer Retinopathie, akuter Lungenschädigung, akutem Atemnotsyndrom, Atherosklerose, choroidalem Ödem, Choroiditis, koronarer mikrovaskulärer Krankheit, zerebraler mikrovaskulärer Krankheit, Eales-Krankheit, verletzungsbedingtem Ödem, mit Hypertonie assoziiertem Ödem, hämorrhagischem Schock, Hypertonie, Irvine-Gass-Syndrom, Ischämie, Makulaödem, Nephritis, Nephropathien, nephrotischem Ödem, nephrotischem Syndrom, Neuropathie, Organversagen aufgrund Ödem, Präeklampsie, Lungenödem, pulmonaler Hypertonie, Nierenversagen, Netzhautödem, Netzhautblutung, Netzhautvenenverschluss, Retinitis, Retinopathie, stummem Hirninfarkt, systemischem Entzündungssyndrom, Transplantations-Glomerulopathie, vaskulärem Lecksyndrom, Glaskörperblutung und Von-Hippel-Lindau-Krankheit, beinhaltend die Verabreichung einer wirksamen Menge des Wirkstoffs an ein Tier, wobei das Diketopiperazin aus der Gruppe, die aus DA-DKP, MR-DKP und YE-DKP besteht, ausgewählt ist.

2. Wirkstoff zur Verwendung gemäß Anspruch 1, wobei mit der Verabreichung des Diketopiperazins oder Salzes unmittelbar nach der Diagnose der Erkrankung oder des Krankheitszustands begonnen wird.

3. Wirkstoff zur Verwendung gemäß Anspruch 1, wobei das Tier das Diketopiperazin oder Salz aufgrund von einem oder mehreren frühen Anzeichen von oder einer Prädisposition zur Entstehung einer Erkrankung oder eines Krankheitszustands, die oder der durch vaskuläre Hyperpermeabilität vermittelt wird, benötigt.

4. Wirkstoff zur Verwendung gemäß Anspruch 3, wobei es sich bei der Erkrankung oder dem Krankheitszustand um ein diabetisches Makulaödem oder eine altersbedingte Makuladegeneration handelt.

5. Wirkstoff zur Verwendung gemäß Anspruch 1, wobei die vaskuläre Hyperpermeabilität eine vaskuläre Hyperpermeabilität eines geschlossenen Endothels ist, das in oder um Gehirn, Diaphragma, Duodenummuskulatur, Fett, Herz, Niere, große Blutgefäße, Lunge, Mesenterium, Nerven, Netzhaut, Skelettmuskeln, Haut oder Hoden zu finden ist.

6. Wirkstoff zur Verwendung gemäß Anspruch 1, wobei es sich bei der vaskulären Hyperpermeabilität um vaskuläre Hyperpermeabilität eines gefensterten Endothels handelt, das in oder um Niere, Bauchspeicheldrüse, Nebenniere, endokrine Drüse oder Darm zu finden ist.

7. Wirkstoff zur Verwendung gemäß einem der Ansprüche 1 bis 6, wobei das Diketopiperazin oder Salz oral verabreicht wird.

8. Wirkstoff zur Verwendung gemäß einem der Ansprüche 1 bis 7, wobei das Tier ein Mensch ist.

## Revendications

1. Ingrédient actif comprenant une dicétopipérazine ou un sel pharmaceutiquement acceptable de celle-ci pour une utilisation dans l'inhibition de l'hyperperméabilité vasculaire chez un animal qui présente une maladie ou une affection sous la médiation d'une hyperperméabilité vasculaire sélectionnée dans le groupe consistant en les suivantes : oedème maculaire diabétique, dégénérescence maculaire liée à l'âge, oedème myocardique, fibrose myocardique, dysfonctionnement diastolique, cardiomyopathie diabétique, retard du développement pulmonaire chez des foetus de mères diabétiques, hyperplasie vasculaire au niveau du mésentère, neuropathie diabétique, néphropathie diabétique, rétinopathie diabétique, lésion pulmonaire aiguë, syndrome de détresse respiratoire aiguë, athérosclérose, oedème de la choroïde, choroïdite, maladie coronaire microvasculaire, maladie cérébrale microvasculaire, maladie d'Eales, oedème secondaire à une lésion, oedème associé à une hypertension artérielle, choc hémorragique, hypertension artérielle, syndrome d'Irvine-Gass, ischémie, oedème maculaire, néphrite, néphropathies, oedème néphrotique, syndrome néphrotique, neuropathie, défaillance d'un organe due à un oedème, prééclampsie, oedème pulmonaire, hypertension pulmonaire, insuffisance rénale, oedème rétinien, hémorragie rétinienne, occlusion veineuse rétinienne, rétinite, rétinopathie, infarctus cérébral silencieux, syndrome de réponse inflammatoire systémique, glomérulopathie du transplant, syndrome de fuite vasculaire, hémorragie du vitré et maladie de Von Hippel-Lindau, qui comprend l'administration à l'animal d'une quantité efficace de l'ingrédient actif, la dicétopipérazine étant sélectionnée dans le groupe consistant en la DA-DKP, la MR-DKP et la YE-DKP.

2. Ingrédient actif pour une utilisation selon la revendication 1, dans laquelle l'administration de la dicétopipérazine ou du sel débute immédiatement au diagnostic de la maladie ou de l'affection.

3. Ingrédient actif pour une utilisation selon la revendication 1, dans laquelle l'animal requiert la dicétopipérazine ou le sel parce qu'il présente un ou plusieurs signes précoces ou une prédisposition à développer une maladie ou affection sous la médiation d'une hyperperméabilité vasculaire.

4. Ingrédient actif pour une utilisation selon la revendication 3, dans laquelle la maladie ou l'affection est un oedème maculaire diabétique ou une dégénérescence maculaire liée à l'âge.

5. Ingrédient actif pour une utilisation selon la revendication 1, dans laquelle l'hyperperméabilité vasculaire est une hyperperméabilité vasculaire d'un endothélium continu présent à l'intérieur ou autour d'un cerveau, d'un diaphragme, d'une musculature duodénale, de graisses, d'un coeur, d'un rein, d'un vaisseau sanguin de gros calibre, d'un poumon, d'un mésentère, d'un nerf, d'une rétine, d'un muscle squelettique, d'une peau ou d'un testicule.

6. Ingrédient actif pour une utilisation selon la revendication 1, dans laquelle l'hyperperméabilité vasculaire est une hyperperméabilité vasculaire d'un endothélium fenestré présent à l'intérieur ou autour d'un rein, d'un pancréas, d'une surrénale, d'une glande endocrine ou d'un intestin.

7. Ingrédient actif pour une utilisation selon l'une quelconque des revendications 1 à 6, dans laquelle la dicétopipérazine ou le sel est administré par voie orale.

8. Ingrédient actif pour une utilisation selon l'une quelconque des revendications 1 à 7, dans laquelle l'animal est un humain.
